# DEMANDE DE BREVET EUROPEEN

(11) **EP 1 857 460 A2**
(43) Date de publication de la demande: **21.11.2007**
(21) Numéro de dépôt: 07108478.4
(22) Date de dépôt: 18.05.2007
(51) Int. Cl.: C07F 5/00, A61K 31/618, A61P 35/00, A61P 31/00, A61P 29/00

(54) **Nouveaux complexes métalliques, procédé pour leur préparation et leur utilisation thérapeutique**

(30) Priorité: 19.05.2006 FR 0651856
(71) Demandeur: SOCIETE DE COORDINATION DE RECHERCHES THERAPEUTIQUES, 20220 Algajola (FR)
(72) Inventeur: Badawi, Abelfattah Mohsen, 11771, Le Caire (EG); Collery, Philippe, 20220, Bastia (FR)
(74) Mandataire: Gillard, Marie-Louise

(57) **Abrégé**

La présente invention a pour objet des complexes métalliques répondant à la formule générale (I) : 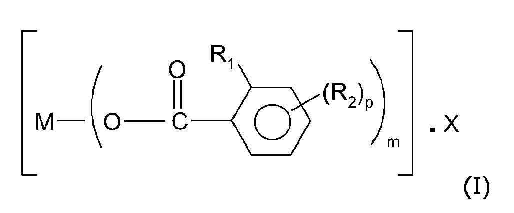 dans laquelle :
- M est un métal ayant un degré d'oxydation égal à 3 ou 4 ;
- R₁ est le groupe hydroxy ou acétyloxy ;
- R₂ est l'hydrogène, le groupe amino, un atome d'halogène ou un groupe (C₁-C₆)alkyle ;
- p est un nombre entier de 1 à 3 ;
- m est un nombre entier égal à 3 ou 4 ;

X est choisi parmi CₙH₂ₙ₊₁ OH or DMSO.

Ces complexes ont des propriétés pharmacologiques intéressantes, notamment pour le traitement des cancers, des maladies infectieuses ou inflammatoires.

## Description

La présente invention a pour objet des nouveaux complexes métalliques qui ont des propriétés pharmacologiques intéressantes, notamment pour le traitement des cancers, des maladies infectieuses ou inflammatoires.

La présente invention concerne également un procédé pour la préparation de ces complexes ainsi que les compositions pharmaceutiques le contenant.

La grande majorité des patients atteints de cancers subissent une chimiothérapie. Le choix judicieux de l'agent anti-tumoral approprié ou du mélange d'agents anti-tumoraux appropriés est un défi continuel face à l'évolution de la réponse de la tumeur au traitement.

On sait que les ions métalliques, tels que les ions de gallium, palladium, ruthénium, rhénium, vanadium et platine, sont des agents antitumoraux efficaces. A cet effet, on peut citer par exemple le brevet EP 0 059 148 qui décrit l'utilisation du chlorure de gallium comme agent anticancéreux ; le brevet EP 0 599 881, qui décrit des complexes du gallium utiles comme agents anticancéreux.

On a maintenant trouvé des nouveaux complexes métalliques qui présentent des propriétés pharmacologiques intéressantes, notamment dans le traitement des cancers, des maladies infectieuses ou inflammatoires.

Les complexes métalliques selon l'invention sont des dérivés de l'acide salicylique qui répondent à la formule générale (I) : 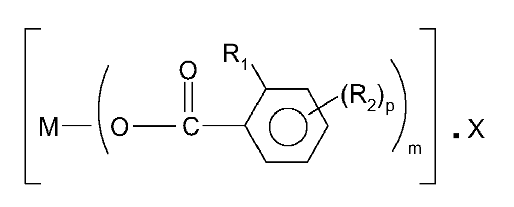 dans laquelle :
- M est un métal ayant un degré d'oxydation égal à 3 ou 4 ;
- R₁ est le groupe hydroxy ou acétyloxy ;
- R₂ est l'hydrogène, le groupe amino, un atome d'halogène ou un groupe (C₁-C₆)alkyle ;
- p est un nombre entier de 1 à 3 ;
- m est un nombre entier égal à 3 ou 4 ;
- X est choisi parmi CₙH₂ₙ₊₁ OH ou DMSO.

De préférence, M est un métal ayant des propriétés thérapeutiques qui est choisi parmi le gallium, le ruthénium, le rhénium, le vanadium, le platine et le palladium.

Parmi les composés de formule (I) ci-dessus, on préfère en particulier ceux dans lesquels X est un alcool en C₂ - C₁₈, de préférence l'éthanol.

Des composés de formule (I) particulièrement préférés de l'invention sont ceux dans lesquels M est le gallium.

Parmi les complexes de gallium de formule (I), on préfère tout particulièrement les composés de formule (Ia) ci-après : 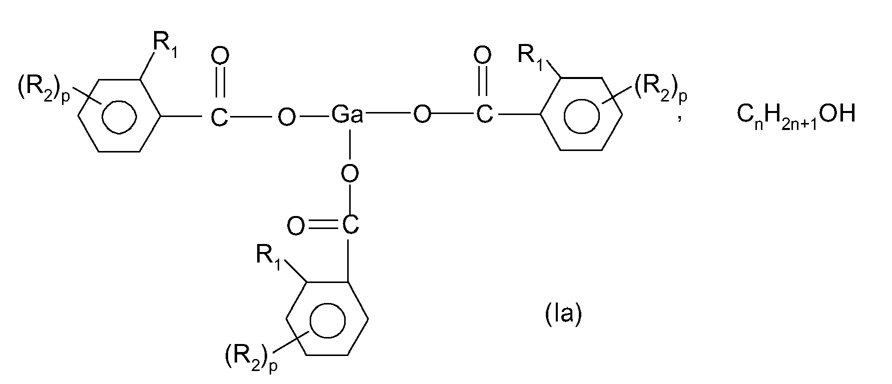 dans laquelle :
- R₁ est le groupe hydroxy ou acétyloxy ;
- R₂ est l'hydrogène, le groupe amino, un atome d'halogène, par exemple le chlore ou un groupe (C₁-C₆)alkyle ;
- p est un nombre entier de 1 à 3 ;
- n est un nombre entier de 2 à 18.

De préférence, R₂ est le groupe NH₂, un atome de chlore ou le groupe isopropyle.

Avantageusement, R₂ est le chlore et p est un nombre entier égal à 1, 2 ou 3 ou bien R₂ est le groupe isopropyle et p est égal à 2.

Le composé particulièrement préféré selon l'invention est le [tris(salicylate) de gallium].éthanol, c'est-à-dire le composé de formule (Ia) dans laquelle R₁ est le groupe hydroxy et R₂ est l'hydrogène, p est 1 et n est égal à 2.

Les composés de l'invention peuvent être obtenus en faisant réagir un hydroxyde métallique de formule M(OH)ₘ dans laquelle m correspond au degré d'oxydation du métal M avec un dérivé approprié de l'acide salicylique en présence d'un alcool CₙH₂ₙ₊₁OH ou de diméthylsulfoxyde (DMSO). Ce procédé est avantageusement réalisé à la température de reflux du mélange.

Les composés de formule (I) ont fait l'objet d'essais pharmacologiques permettant de déterminer ses activités à la fois sur les cellules malignes en culture et sur les tumeurs inoculées chez des animaux.

Les mesures de ces activités ont été effectuées en utilisant les tests ci-après :
- de culture de cellules EAC selon la méthode de El-MERZABANI et al. (Screening system for Egyptian plants with potential antitumor activity. J.Planta Medica,36:150-155).
- de survie d'animaux porteurs de tumeurs.

Compte-tenu de l'activité de ces nouveaux dérivés ils peuvent être utilisés en thérapeutique notamment comme agents anticancéreux.

Ainsi selon un autre aspect, l'invention concerne également les compositions pharmaceutiques contenant à titre de principe actif un composé de formule (I) en combinaison avec au moins un véhicule pharmaceutiquement acceptable.

Les véhicules pharmaceutiquement acceptables sont choisis en fonction du mode d'administration utilisé pour le traitement envisagé, en particulier par voie parentérale ou orale, l'administration orale étant préférée, notamment sous forme d'ampoules buvables.

Les composés selon l'invention peuvent être administrés à des doses variables en fonction de l'état du patient et de la tumeur à traiter. Ces doses peuvent être par exemple de 1 à 100 mg par kg de poids du patient et par jour.

L'invention va maintenant est décrite plus en détails par les exemples illustratifs ci-après.

### EXEMPLE 1 : Synthèse du [tris(salicylate) de gallium].éthanol.

De l'hydroxyde de gallium Ga(OH)₃ (0.97 mg) a été ajouté à de l'acide salicilyque (6,98 mg) dissout dans 150 ml d'éthanol anhydre. La suspension ainsi obtenue a été portée au reflux sur un bain-marie jusqu'à la réaction complète de l'hydroxyde de gallium. La solution blanchâtre résultante a ensuite été filtrée et les cristaux blancs ont été récupérés par refroidissement. Ils ont été essorés puis lavés avec de l'éthanol froid et séchés à l'air et on a ainsi obtenu 3,2 mg de [tris(salicylate) de gallium].éthanol qui présentaient les caractéristiques ci-après :

### Spectre IR :

CO à 1703 cm⁻¹ ;
n-OH à 1300 cm⁻¹ ;
n-OH aliphatique à 3650-3590 cm⁻¹ ;
n-CH aliphatique 2920 cm⁻¹ ;
CH aromatique à 3047 cm⁻¹.

### Spectre en masse :

Pic à m/e 120 (100%) attribué à OH-(C₆H₅) C⁺O ;
Pic à m/e 69 dû à l'ion gallium.

### Spectre

RMN H⁺ (CDCl₃) :
2s (OH) alccol ; 3,6 t₂ (CH₂);
5s (OH) aromatique ; 7,4-7,8 m (5H ; aryl)
s=singulet ; t= triplet ; m=multiplet

### Microanalyse

| Composé | | Microanalyse % | | |
|---|---|---|---|---|
| | | C | H | Ga |
| [tris(salicylate) de gallium].éthanol | Calculé | 52,5 | 3,8 | 13,25 |
| | Trouvé | 52,27 | 3,68 | 13,00 |

### EXEMPLE 2 : Effet inhibiteur sur les cellules malignes en culture.

L'effet du [tris(salicylate) de gallium].éthanol sur la viabilité de cellules EAC a été étudié selon la méthode El-Merzabani et al. Indiquée précédemment.

Dans la présente description, on désigne par « cellules EAC » des cellules du carcinome ascitique d'Ehrlich.

Dans cet essai, le [tris(salicylate) de gallium].éthanol a été utilisé à des doses allant de 0,8 à 100 mg par ml. L'effet de cette différente concentration de salicylate de gallium sur la survie des cellules EAC cultivées est représenté dans le tableau 2.

**Tableau 2 : Effet des différentes concentrations de [tris(salicylate) de gallium].éthanol sur la viabilité des cellules EAC.**

| Concentrations de [tris(salicylate) de gallium].éthanol | Pourcentage de cellules non viables X±SD* (valeurs extrêmes) |
|---|---|
| 0 | 1% |
| 0,8 | 13,7 ± 1,8 (a) (11-16) |
| 1,6 | 26,3 ± 2,1 (a) (24-29) |
| 3,2 | 41,0 ± 1,3 (a) (39-42) |
| 6,3 | 50,2 ± 1,5 (a) (48-52) |
| 12,5 | 57,0 ± 0,89 (a) (56-58) |
| 25,0 | 64,0 ± 1,5 (a) (62-66) |
| 50,0 | 69 ± 1,5 (a) (67-71) |
| 100,0 | 72,3 ± 1,8 (a) (70-73) |

| | |
|---|---|
| (a) significatif à p<0,001 - exprimé en % de cellules non viables * moyenne ± SD pour 6 expériences | |

Ces résultats sont exprimés en pourcentage de cellules non viables. On constate que l'addition de [tris(salicylate) de gallium].éthanol au milieu de cultures de cellules EAC provoque un effet cytotoxique qui dépend de la dose. Cet effet cytotoxique varie de 13,7% de cellules non viables pour une concentration de 0,8 mg de [tris(salicylate) de gallium].éthanol par ml à 72,3 % de cellules non viables lorsque le [tris(salicylate) de gallium].éthanol est utilisé à une concentration de 100 mg par ml.

### EXEMPLE 3 : Effet anti-tumoraux sur des animaux porteurs d'une tumeur

Dans cet exemple, on a utilisé 30 souris femelles albinos pesant 20 g chacune. Ces souris ont été divisées en trois groupes :
- Groupe 1 : Groupe contrôle. C'est-à-dire les souris ne recevant aucun traitement.
- Groupe 2 : Groupe des souris porteuses de tumeur. On a injecté à ces souris par voie intra parentérale 10,5x10⁶ cellules EAC.
- Groupe 3 : Les souris ont reçu par injection intra parentérale 2,5 x10⁶ cellules EAC et ensuite ont été traitées à la dose de 50 mg par kg de leur poids, de [tris(salicylate) de gallium].éthanol aux jours 2, 4 et 6 après l'inoculation de la tumeur.

La survie des souris a été comparée dans les trois groupes.

### Poids des souris :

L'effet du [tris(salicylate) de gallium].éthanol sur le poids des souris porteuses de cellules EAC a été calculé comme le pourcentage de changements du poids à partir du poids du premier jour.

### Survie des animaux :

La survie des souris dans les différents groupes a été estimée continuellement pendant une période de 40 jours. 30% des souris inoculées avec des cellules EAC et traitées avec du [tris(salicylate) de gallium].éthanol ont survécu pendant 30 jours.

## Revendications

1. Complexes métalliques répondant à la formule générale (I) : 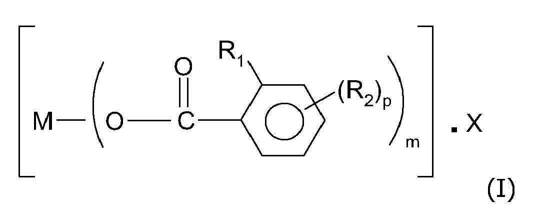 dans laquelle :
- M est un métal ayant un degré d'oxydation égal à 3 ou 4 ;
- R₁ est le groupe hydroxy ou acétyloxy ;
- R₂ est l'hydrogène, le groupe amino, un atome d'halogène ou un groupe (C₁-C₆)alkyle ;
- p est un nombre entier de 1 à 3 ;
- m est un nombre entier égal à 3 ou 4 ;
- X est choisi parmi CₙH₂ₙ₊₁ OH or DMSO.

2. Complexes selon la revendication 1, **caractérisés en ce que** M est choisi parmi le gallium, le ruthénium, le rhénium, le vanadium, le platine et le palladium.

3. Complexes selon l'une des revendications 1 ou 2, **caractérisés en ce que** M est le gallium.

4. Complexes selon l'une quelconque des revendications 1 à 3, **caractérisés en ce que** X est un alcool de formule CₙH₂ₙ₊₁ OH dans laquelle n est un nombre entier de 2 à 18.

5. Complexes selon la revendication 4, **caractérisés en ce que** l'alcool de formule CₙH₂ₙ₊₁ OH est l'éthanol.

6. Complexes selon l'une quelconque des revendications 1 à 5, **caractérisés en ce qu'**ils répondent à la formule (Ia) : 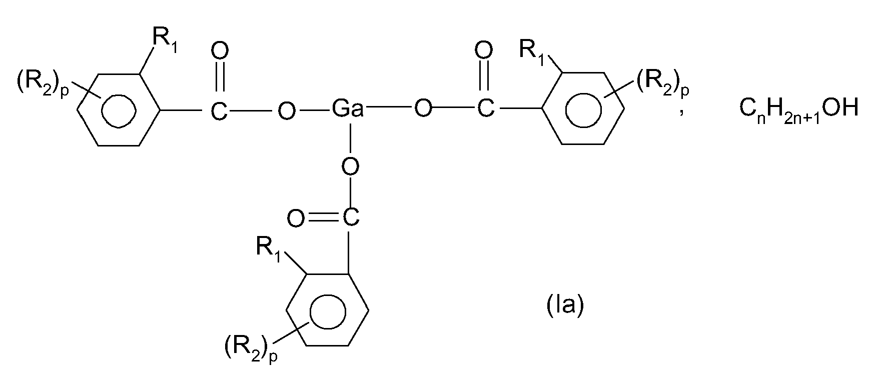 dans laquelle :
- R₁ est le groupe hydroxy ou acétyloxy ;
- R₂ est l'hydrogène, le groupe amino, un atome d'halogène ou un groupe (C₁-C₆)alkyle ;
- p est un nombre entier de 1 à 3 ;
- n est un nombre entier de 2 à 18.

7. Procédé de préparation d'un complexe métallique selon la revendication 1, **caractérisé en ce qu'**il consiste à faire réagir un hydroxyde métallique de formule M(OH)ₘ dans laquelle m correspond au degré d'oxydation du métal M avec un dérivé de l'acide salicylique en présence d'un alcool de formule CₙH₂ₙ₊₁ OH ou DMSO.

8. Composition pharmaceutique contenant un complexe métallique selon l'une quelconque des revendications 1 à 6 à titre de principe actif en combinaison avec un véhicule pharmaceutique acceptable.

9. Composition pharmaceutique selon la revendication 8 **caractérisée en ce que** le principe actif est le [tris(salicylate) de gallium].éthanol.
